Europäisches Patentamt

European Patent Office

Office européen des brevets

(11) Veröffentlichungsnummer: **0 292 803**
**A2**

(12) **EUROPÄISCHE PATENTANMELDUNG**

(21) Anmeldenummer: 88107661.6

(22) Anmeldetag: 13.05.88

(51) Int. Cl.⁴: **C12N 9/04 , C12N 15/00 , C12N 1/20 , C07K 7/10 , //(C12N1/20,C12R1:19)**

Patentanspruch für folgenden Vertragsstaat: ES.

(30) Priorität: 23.05.87 DE 3717437

(43) Veröffentlichungstag ... ~r Anmeldung:
30.11.88 Patentblatt 88/48

(84) Benannte Vertragsstaaten:
AT BE CH DE ES FR GB GR IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Uhlmann, Eugen, Dr.**
**Zum Talblick 31**
**D-6246 Glashütten/Taunus(DE)**
Erfinder: **Hein, Friedrich, Dr.**
**Hufnagelstrasse 13**
**D-8000 München 21(DE)**

(54) **Verfahren zur Herstellung eines HMG-CoA-Reduktase-ähnlichen Proteins.**

(57) Das Gen für die aktive Domäne (461 carboxyterminale Aminosäuren) der humanen 3-Hydroxy-3-methylglutaryl-Coenzym A-Reduktase wird (in Form von 4 Fragmenten aus 24 Oligonukleotiden) chemoenzymatisch synthetisiert. Es erlaubt die gentechnische Herstellung des entsprechenden Proteins.

EP 0 292 803 A2

### Verfahren zur Herstellung eines HMG-CoA-Reduktase-ähnlichen Proteins

3-Hydroxy-3-methylglutaryl-Coenzym A-Reduktase (HMG-CoA-Reduktase) ist das geschwindigkeitsbestimmende Enzym der Cholesterin-Biosynthese (Brown und Goldstein (1980) J. Lipid Res. 21, 505 - 517). Die HMG-CoA-Reduktase des Hamsters ist ein 97 kD Glykoprotein (887 Aminosäuren), das durch eine hydrophobe aminoterminale Domäne (339 Aminosäuren) an die Membran des endoplasmatischen Retikulums gebunden ist (Chin et al. (1984) Nature 308, 613 - 617; Liscum et al. (1985) J. Biol. Chem. 260, 522 - 530). Die carboxyterminalen wasserlösliche Domäne (548 Aminosäuren) ist ins Cytoplasma gerichtet und enthält das aktive Zentrum des Enzyms. Während diese beiden Domänen beim Vergleich der Hamster- und Human-Sequenz stark konserviert sind (Luskey und Stevens (1985) J. Biol. Chem. 260, 10271 - 10277), divergiert die die Domänen verbindende Spacer-Region stärker. Die menschliche HMG-CoA-Reduktase ist aus 888 Aminosäuren aufgebaut.

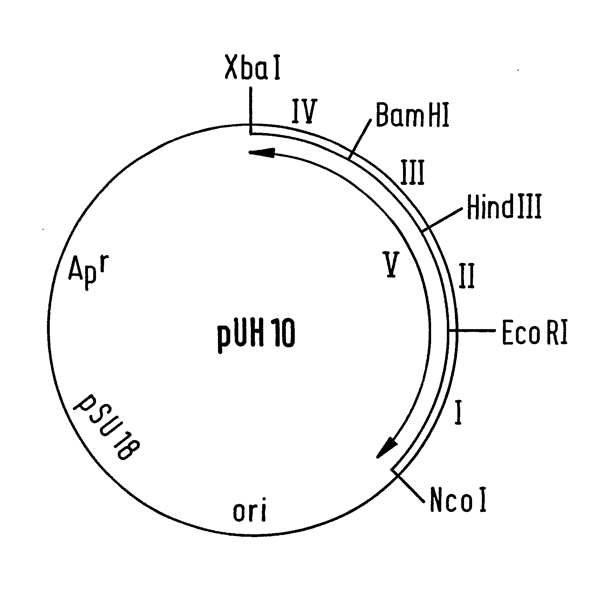

Die vorliegende Erfindung betrifft ein Verfahren zur Herstellung eines Gens für die aktive Domäne der humanen HMG-CoA-Reduktase (Aminosäuren 428 - 888 aus Fig. 2 bei Luskey und Stevens), also für die 461 carboxyterminalen Aminosäuren der bekannten Sequenz. Diese Teilsequenz wird im folgenden "HMG-CoA-Reduktase-ähnliches Protein" genannt. Ferner betrifft die Erfindung ein Verfahren zur Amplifikation und Expression des Gens für das HMG-CoA-Reduktase-ähnliche Protein. Das HMG-CoA-Reduktase-ähnliche Protein besitzt die volle enzymatische Aktivität und wird durch die HMG-CoA-Reduktase-Hemmer Mevinolin, Compactin sowie synthetische Derivate derselben in gleicher Weise wie das Gesamtprotein inhibiert. Üblicherweise werden HMG-CoA-Reduktase-Hemmer zunächst an tierischen Enzympräparationen getestet. Durch die gentechnische Herstellung des HMG-CoA-Reduktase-ähnlichen Proteins steht nun ein humanes Protein mit identischer enzymatischer Aktivität wie die humane HMG-CoA-Reduktase in ausreichender Menge zur Verfügung. Ferner ist das "Design" des synthetischen Gens mit den zahlreichen Schnittstellen besonders gut zur Herstellung mutanter HMG-CoA-Reduktase-ähnlicher Proteine geeignet. Auf diese Weise hat man ein wertvolles Werkzeug zur Untersuchung der Struktur/Aktivitäts-Beziehung von Enzym und Enzymhemmer zur Hand. Dadurch werden die Wechselwirkungen auf molekularer Ebene verständlicher, wodurch wiederum effizientere Hemmer entworfen und synthetisiert werden können.

Der genetische Code ist bekanntlich "entartet", d. h. daß nur für zwei Aminosäuren eine einzige Nukleotidsequenz codiert, während den restlichen 18 codierbaren Aminosäuren zwei bis sechs Tripletts zuzuordnen sind. Für die Synthese des Gens steht somit eine große Vielfalt von Codon-Kombinationen zur Auswahl. Es wurde nun gefunden, daß die DNA-Sequenz V (Tab. 5) besonders vorteilhaft ist.

Am 5'-Ende des codierenden Stranges befindet sich eine "überhängende" DNA-Sequenz, entsprechend der Restriktionsendonuklease NcoI, die zugleich das Translations-Startcodon (ATG) beinhaltet. Am 3'-Ende des codierenden Stranges ist dagegen die einzelsträngige Sequenz entsprechend dem Restriktionsenzym XbaI überhängend. Diese beiden unterschiedlichen Erkennungssequenzen gewährleisten die Insertion der DNA in Plasmide in der gewünschten Orientierung. Der codierenden Sequenz folgen auf das Triplett Nr. 461 für Alanin zwei Translations-Terminationscodons (Stop-Codons).

Drei interne singuläre Schnittstellen für die Restriktionsenzyme EcoRI (Codon 123/124), HindIII (Codon 243/244) und BamHI (Codon 346/347) ermöglichen die Subklonierung der Genfragmente HMG I, HMG II, HMG III und HMG IV (DNA-Sequenzen I - IV, Tab. 1 - 4), die in gut untersuchte Plasmide wie pUC18 oder Derivate dieser Plasmide, die sich nur im Polylinkerteil unterscheiden, eingebaut werden können.

Die Figur zeigt das Plasmid pUH10, das das Gesamtgen im pUC18-Derivat pSU18 enthält. I - IV bezeichnen die Fragmente HMG I - IV.

Das Genfragment HMG II kann über die Schnittstellen MluI und BssHII weiter in die Subklone HMG II-1, HMG II-2 und HMG II-3 unterteilt werden. Diese Fragmente sind in den DNA-Sequenzen II-1 bis II-3 (Tab. 2.1 - 2.3) enthalten, welche über temporäre Schnittstellen in kommerziell erhältliche Plasmide wie pUC18 eingebaut werden. Die zur Konstruktion des Genfragmentes HMG II notwendigen Gensubfragmente lassen sich durch Verdauung mit den entsprechenden Restriktionsenzymen gewinnen.

Das Genfragment HMG III läßt sich aufgrund der internen Schnittstellen für die Restriktionsenzyme Sau96I und BanII weiter in die Subklone HMG III-1, HMG III-2 und HMG III-3 aufteilen. Diese Fragmente sind in den DNA-Sequenzen III-1 bis III-3 (Tab. 3.1 - 3.3) enthalten und können aus diesen durch Verdauung mit den entsprechenden Restriktionsenzymen gewonnen werden.

Das Genfragment IV kann über die temporäre Schnittstelle für das Restriktionsenzym EcoRI in zwei Subklone HMG IV-(1+2) und HMG IV-(3+4) unterteilt werden. Die zusätzlich erforderliche Nukleotidfolge AATT, die in der DNA-Sequenz IV nicht enthalten ist, kann nach Kombination der beiden Subklone HMG IV-(1+2) und HMG IV-(3-4) in bekannter Weise (Chakhmakhcheva et al. (1986) Chemica Scripta 26, 31) mit S1-Exonuklease oder "mung bean" Nuklease wieder entfernt werden.

Zusätzlich wurden innerhalb des Strukturgens eine Reihe von weiteren singulären Erkennungssequenzen für Restriktionsenzyme eingebaut, die einerseits einen Zugang zu Teilsequenzen des HMG-CoA-Reduktase-ähnlichen Proteins schaffen und andererseits die Durchführung von Mutationen und Variationen wie Einbau, Austausch oder Deletion von Nukleotiden erlauben:

| Restriktionsenzym | Schnitt nach Nukleotid Nr. (codierender Strang) |
|---|---|
| AccI | 66 |
| AflIII | 488 |
| Aha II | 113 |
| Asp 718 | 1130 |
| BalI | 1015 |
| BclI | 573 |
| BglI | 1289 |
| BglII | 671 |
| BsmI | 136 |
| BssHII | 608 |
| BstEII | 10 |
| Cfr10I | 1265 |
| ClaI | 199 |
| DraI | 883 |
| EagI | 1296 |
| EcoO109 | 346 |
| EcoRV | 700 |
| HaeII | 1364 |
| HgiAI | 241 |
| KpnI | 1130 |
| MluI | 488 |
| MstII | 1071 |
| NaeI | 1265 |
| NciI | 1227 |
| NdeI | 1319 |
| NheI | 413 |

| Restriktionsenzym | Schnitt nach Nukleotid Nr. (codierender Strang) |
|---|---|
| NruI | 265 |
| NspHI | 1199 |
| PpuMI | 346 |
| PstI | 528 |
| PvuI | 438 |
| PvuII | 1102 |
| SacI | 241 |
| SacII | 38 |
| SfaNI | 957 |
| SpeI | 629 |
| SphI | 1199 |
| SspI | 917 |
| SstII | 38 |
| StuI | 1167 |
| XhoI | 164 |
| XmaIII | 1296 |

Die DNA-Sequenz V wurde in wesentlichen Punkten von der bekannten natürlichen Human-Sequenz abgeändert. Dadurch war die Einführung der zahlreichen singulären Schnittstellen für Restriktionsenzyme möglich. Ferner konnten dadurch palindromische Sequenzen, die keine Erkennungsstelle für Restriktionsenzyme besitzen, aber bei der Gen-Synthese störend wirken, auf ein Mindestmaß reduziert werden. Die DNA-Sequenz V läßt sich aus insgesamt 24 Oligonukleotiden mit einer Kettenlänge von 58 bis 147 Nukleotideinheiten aufbauen. Dabei verfährt man wie nachfolgend beschrieben.

Das Genfragment HMG I, entsprechend der DNA-Sequenz I, läßt sich aus 10 Oligonukleotiden mit einer Kettenlänge von 64 bis 82 Einheiten aufbauen, indem diese zunächst chemisch synthetisiert und dann über "sticky ends" von 12 Nukleotiden enzymatisch verknüpft werden.

Das Genfragment HMG II, entsprechend der DNA-Sequenz II, ist aus sechs chemisch synthetisierten Oligonukleotiden mit einer Länge von 120 bis 136 Nukleotideinheiten zu erhalten. Dabei lassen sich die Vorläufer der Genfragmente HMG II-1 bis HMG II-3 (Tabl. 2.1 - 2.3), aus jeweils zwei chemisch synthetisierten Oligonukleotiden aufbauen. Nach der Klonierung und Amplifikation dieser Fragmente können durch Restriktionsenzymverdauung mit EcoRI und MluI für HMG II-1; MluI und BssHII für HMG II-2 sowie BssHII und HindIII für HMG II-3 die zum Aufbau von HMG II (DNA-Sequenz II) notwendigen Subfragmente isoliert werden, die dann schließlich über ihre sticky ends zu HMG II verknüpfbar sind.

Das Genfragment HMG III (DNA-Sequenz III) ist aus nur drei chemisch synthetisierten Oligonukleotiden mit einer Länge von 130, 137 bzw. 147 Einheiten zugänglich. Diese Oligonukleotide besitzen als ein besonderes Charakteristikum an ihrem 3'-Ende eine kurze invertierte Wiederholung ihrer Sequenz. Dies führt dazu, daß sich das Oligonukleotid an seinem 3'-Ende zurückfaltet und eine sogenannte "hairpin"-Struktur annimmt. Das so hybridisierte 3'-Ende dient nun als "Primer" für die DNA-Polymerase (Klenow) oder Amv-Reverse Transcriptase katalysierte Bildung des zweiten Stranges. Die gebildeten hairpin-Strukturen, entsprechend den DNA-Sequenzen III-1 bis III-3, eignen sich noch nicht für eine Verknüpfung mit einem weiteren DNA-Fragment, können jedoch durch Restriktionsenzym-Verdauung (HindIII, EcoRI für DNA-Sequenz III-1, Ban II, Sau 96I für die DNA-Sequenz III-2; EcoRI, BamHI für die DNA-Sequenz III-3) in eine geeignete sticky end-Form übergeführt werden. Nach der Klonierung und Amplifikation, sowie Reisolierung durch Restriktionsenzymverdauung (HindIII, Sau96I für HMG III-1; BanII, BamHI für HMG III-3) werden die Gensubfragmente HMG III-1 und III-3 mit dem nicht subklonierten synthetischen Fragment HMG III-2 (Sau 96I, Ball-sticky ends) zum Genfragment HMG III verknüpft.

4

Das Genfragment IV läßt sich aus 5 (bzw. 4, wenn eine RNA-Ligase-Reaktion durchgeführt wird) Oligonukleotiden mit einer Länge von 58 bis 121 Einheiten aufbauen. Zur Konstruktion des Subfragments HMG IV-(1+2), sind zwei Oligonukleotide, die an ihrem 3'-Ende eine hairpin-Struktur besitzen, chemisch zu synthetisieren und in einer DNA-Polymerase-Reaktion in die doppelsträngige Form überzuführen. Dann werden die Genfragmente, entsprechend der DNA-Sequenz IV-1 bzw. DNA-Sequenz IV-2, mit den Restriktionsenzymen BamHI bzw. EcoRI geschnitten und in ein mit den Restriktionsenzymen EcoRI und BamHI geöffnetes Plasmid eingebaut. Nach der Ligation werden die Enden mit dem Restriktionsenzym KpnI oder Asp718 nachgeschnitten und das Plasmid anschließend mit T4 DNA-Ligase rezirkuliert. Zum Aufbau des Subfragments HMG IV-(3+4), sind drei Oligonukleotide chemisch zu synthetisieren. Das 121-mer Oligonukleotid HMG IV-4a besitzt an seinem 3'-Ende eine hairpin-Struktur und wird als 5'-Phosphat synthetisiert. Das 86-mer Oligonukleotid HMG IV-3a besitzt an seinem 3'-Ende eine Ribonukleosid-Einheit, im speziellen Falle also einen Cytidin-Rest. Damit kann es in an sich bekannter Weise (Brennan et al., Nucleic Acids' Res. (1985) 13, 8665) mit Hilfe von T4 RNA-Ligase mit dem Oligonukleotid-5'-Phosphat HMG IV-4a verknüpft werden. Die beiden Oligonukleotide können jedoch auch mit Hilfe des komplementären 58-mer Oligonukleotid-5'-phosphats HMG IV-3b in einer T4 DNA-Ligase-katalysierten Reaktion verknüpft werden. Die Bildung des DNA-Doppelstranges erfolgt im ersten Falle mit AMV-Reverse Transcriptase und dNTP, im zweiten Falle mit DNA-Polymerase (Klenow) und dNTP in Gegenwart von DNA-Ligase. Nach Restriktionsenzymverdauung mit EcoRI und XbaI kann das Genfragment HMG IV-(3+4) in ein entsprechend mit EcoRI und XbaI geöffnetes Plasmid eingebaut werden. Die beiden Fragmente HMG IV-(1+2) und HMG IV-(3+4) werden nach Amplifikation und Reisolation wie oben beschrieben unter Eliminierung des Tetranukleotids AATT zum Genfragment HMG IV verknüpft.

Zur Subklonierung der Genfragmente HMG I bis HMG IV und zur Konstruktion des Gesamtgens werden spezielle Plasmide hergestellt. Diese leiten sich von den käuflichen Vektoren pUC18, pUC19 bzw. M13mp18 oder M13mp19 ab, wobei die Polylinker-Region durch einen neuen synthetischen Polylinker, entsprechend der DNA-Sequenz VI

```
        NcoI              EcoRI    HindIII  BamHI    XbaI

                                  VI-1a

 5' AAT TGC CAT GGG CAT GCG GAA TTC CAA GCT TTG GAT CCA TCT AGA GGG

 3'        CG GTA CCC GTA CGC CTT AAG GTT CGA AAC CTA GGT AGA TCT CCC TCG A

                                  VI-1b
```

ersetzt wurde.

Diese neuen Plasmide haben den Vorteil, daß sie im Gegensatz zu den pUC- bzw. M13mp-Plasmiden die Klonierung von DNA-Fragmenten mit den überhängenden Sequenzen für das Restriktionsenzym NcoI erlauben. Zudem sind die Erkennungssequenzen für die Schnittstellen NcoI, EcoRI, HindIII, BamHI und XbaI in den Vektoren genau in der Reihenfolge enthalten, wie sie im Gesamtgen HMG vorliegen, was die sequentielle Klonierung und die Konstruktion dieses Gens erleichtert. Somit können die Genfragmente HMG I bis HMG IV in den neuartigen Plasmiden subkloniert werden. Nach Amplifikation der Genfragmente können diese, wie im Beispiel 9 beschrieben, zum Gesamtgen kombiniert werden.

Die Genstruktur der DNA-Sequenz V ist somit aus nur 24 Oligonukleotiden zugänglich, ermöglicht die Subklonierung von 11 Genfragmenten in gut bekannte Vektoren und erlaubt deren Kombination zum Gesamtgen sowie Veränderungen derselben.

Mit Hilfe der überhängenden Enden kann das Gesamtgen in entsprechende Expressionsvektoren eingebaut werden. Neben der Direktexpression des HMG-CoA-Reduktase-ähnlichen Proteins in Bakterien-, Hefe-, Insekten- oder tierischen Zellen, wobei die Nukleotidfolge ATG des NcoI-Überhanges als Startcodon dient, kommt vor allem die Transportexpression (Deutsche Offenlegungsschrift 3 436 818, EP-A2 0 177 827, veröffentlichte australische Patentanmeldung 48 333/85) in Betracht. In E. coli verwendet man vorzugsweise die Signalsequenz der Alkalischen Phosphatase, während in Hefe das $\alpha$-Faktor-System (Zsebo et al. (1986) J. Biol. Chem. 261, 5858 -5865) gute Ergebnisse liefert. Die Expression in tierischen Zellen, beispielsweise CHO-Zellen kann in an sich bekannter Weise, wie für die Hamster-Partialsequenz (Deletionsmutante $\Delta$ $Gly_{10}$ - $Gln_{341}$) beschrieben (Gill et al. (1985) Cell 41, 249 - 258), erfolgen. Der Aktivitätsnachweis ist in derselben Veröffentlichung angegeben.

Die erfindungsgemäß erhaltenen DNA-Sequenzen I - V mit den entsprechenden Subfragmenten, die damit erhaltenen Hybridplasmide, die transformierten Wirtsorganismen sowie das exprimierte HMG-CoA-Reduktase-ähnliche Protein sind ebenfalls Gegenstand der Erfindung.

In den folgenden Beispielen werden einige Ausgestaltungen der Erfindung im einzelnen erläutert. Prozentangaben beziehen sich hierbei auf das Gewicht, wenn nichts anderes angegeben ist.

**Beispiele**

1. Chemische Synthese eines einzelsträngigen Oligonukleotids

Am Beispiel des Oligonukleotids HMG I-1a wird die Synthese der DNA-Bausteine erläutert. Für die Festphasensynthese wird das am 3'-Ende stehende Nukleosid, im vorliegenden Falle also Adenin (Nukleotid Nr. 74) über die 3'-Hydroxyfunktion kovalent an einen Träger gebunden verwendet. Trägermaterial ist mit langkettigen Aminoalkylresten funktionalisiertes CPG ("Controlled Pore Glass").

In den folgenden Syntheseschritten wird die Basenkomponente als 5'-O-Dimethoxytritylnukleosid-3'-phosphorigsäure-ß-cyanoethylester-dialkylamid eingesetzt, wobei das Adenin als $N^6$-Benzoyl-Verbindung, das Cytosin als $N^4$-Benzoyl-Verbindung, das Guanin als $N^2$-Isobutyryl-Verbindung und das Thymin ohne Schutzgruppe vorliegen.

25 mg des polymeren Trägers, der 0,2 μmol 5'-O-Dimethoxytrityl-$N^6$-Benzoyl-2'-desoxyadenosin gebunden enthält, werden nacheinander mit folgenden Agentien behandelt:

A) Acetonitril

B) 3 % Trichloressigsäure in Dichlormethan

C) Acetonitril

D) 5 μmol des entsprechenden Nukleosid-3'-O-phosphits und 25 μmol Tetrazol in 0,15 ml wasserfreiem Acetonitril

E) Acetonitril

F) 20 % Acetanhydrid in Tetrahydrofuran mit 40 % Lutidin und 10 % Dimethylaminopyridin

G) Acetonitril

H) 3 % Jod in Lutidin/Wasser/Tetrahydrofuran im Volumenverhältnis 10 : 1 : 40

Unter "Phospit" wird hierbei der 2'-Desoxyribose-3'-monophosphorigsäure-mono-ß-cyanoethylester verstanden, wobei die dritte Valenz durch einen Diisopropylaminorest abgesättigt ist. Die Ausbeuten der einzelnen Syntheseschritte können jeweils nach der Detritylierungsreaktion B) spektrophotometrisch durch Messung der Absorption des Dimethoxytritylkations bei der Wellenlänge von 496 nm bestimmt werden.

Nach abgeschlossener Synthese erfolgt die Abspaltung der Dimethoxytritylgruppe wie in A) bis C) beschrieben. Durch die Behandlung mit Ammoniak wird das Oligonukleotid vom Träger gespalten und zugleich werden die ß-Cyanoethyl-Gruppen eliminiert. Eine 16-stündige Behandlung der Oligomeren mit konzentriertem Ammoniak bei 50° C spaltet die Aminoschutzgruppen der Basen quantitativ ab. Das so erhaltene Rohprodukt wird durch Polyacrylamid-Gelelektrophorese gereinigt.

2. Chemische 5'-O-Phosphorylierung eines trägergebundenen Oligonukleotides

Das entsprechende Oligonukleotid wird wie in Beispiel 1 beschrieben synthetisiert. Vor der Abspaltung mit conc. Ammoniak behandelt man mit folgenden Reagenzien:

A) Acetonitril

B) 10 μmol Phosphorigsäure-bis-(ß-cyanoethylester)-N,N-diisopropylamid und 25 μmol Tetrazol in 0,15 ml wasserfreiem Acetonitril/Tetrahydrofuran (2 : 1 = v : v)

C) Acetonitril

D) 3 % Jod in Lutidin/Wasser/Tetrahydrofuran (10 : 1 : 40 = -v : v : v)

Anschließend erfolgt die Abspaltung des Oligonukleotids vom Träger mit conc. Ammoniak und die weitere Aufarbeitung wie in Beispiel 1 beschrieben.

3. Enzymatische Verknüpfung der einzelsträngigen Oligonukleotide zum Genfragment HMG I

Zur enzymatischen Phosphorylierung der Oligonukleotide am 5'-Terminus werden je 1 nmc ⁻er Oligonukleotide 1b bis 5a mit 5 nmol Adenosintriphosphat mit vier Einheiten T4 Polynukleotid-Kinase ⁙ 20 µl 50 mM Tris-Hcl-Puffer (pH 7,6), 10 mM Magnesiumchlorid und 10 mM Dithiothreitol (DTT) 30 Minuten bei 37° C behandelt. Das Enzym wird durch fünfminütiges Erhitzen auf 95° C desaktiviert. Die Oligonukleotide 1a und 5b, welche in der DNA-Sequenz I die "überhängenden" einzelsträngigen Sequenzen bilden, werden nicht phosphoryliert. Dies verhindert bei der nachfolgenden Ligation die Ausbildung größerer Genfragmente als sie der DNA-Sequenz I entsprechen.

Die Oligonukleotide 1a bis 5b werden wie folgt zum Genfragment HMG I ligiert: Je 1 nmol der Oligonukleotide 1a und 1b bis 5a und 5b werden paarweise hybridisiert, indem diese in jeweils 20 µl 50 mM Tris-Hcl-Puffer (pH 7,6) 10 mM Magnesiumchlorid und 10 mM DTT gelöst werden, diese Lösung 5 Minuten auf 95° C erhitzt und binnen 2 Stunden auf Raumtemperatur abgekühlt wird. Dabei werden die Oligonukleotide 1b bis 5a in Form ihrer 5'-Phosphate eingesetzt. Zur weiteren Verknüpfung der gebildeten bihelikalen DNA-Fragmente werden die Lösungen derselben vereinigt, 15 Minuten auf 60° C erwärmt und auf Raumtemperatur abgekühlt. Dann setzt man 2 µl 0,1 M DTT, 16 µl 2,5 mM Adenosintriphosphat (pH 7) sowie 1 µl T4 DNA-Ligase (400 units) zu und inkubiert 16 Stunden bei 22° C.

Die Reinigung des Genfragments HMG I erfolgt durch Gelelektrophorese auf einem 6 %-igen Polyacrylamidgel (ohne Harnstoffzusatz, 40 x 20 x 0,1 cm), wobei als Markersubstanz φX174 DNA (Fa. BRL), geschnitten mit Hinfl, oder pBR322, geschnitten mit HaeIII, dient.

4. Hybridisierung von einzelsträngigen synthetischen Oligonukleotiden zu einem klonierbaren Subfragment. Herstellung des Gensubfragments HMG II-1

Je 100 pmol des Oligonukleotids HMG II-1a und des Oligonukleotids HMG II-1b werden zusammen in 50 µl 50 mM Tris-HCl-Puffer (pH 7,6), 10 mM Magnesiumchlorid und 10 mM DTT gelöst, diese Lösung 5 Minuten auf 95° C erhitzt und binnen 2 Stunden auf Raumtemperatur abgekühlt. Der hybridisierte DNA-Doppelstrang, entsprechend der DNA-Sequenz II-1, wird durch Gelchromatographie gereinigt. Auf einem 4 %-igen Agarosegel kann die Größe des gebildeten DNA-Fragments kontrolliert werden, wobei als Marker pBR322, geschnitten mit HaeIII, dient. Dieses Fragment mit den überhängenden Enden für die Restriktionsenzyme EcoRI und SacI wird in bekannter Weise in das mit EcoRI/SacI geöffnete pUC18-Plasmid eingebaut. Man erhält ein Hybridplasmid, enthaltend das Gensubfragment HMG II-1. In analoger Weise lassen sich die Hybridplasmide herstellen, die die Gensubfragmente HMG II-2 und II-3 enthalten.

5. DNA-Doppelestrang-Bildung und Erzeugung überhängender Enden, ausgehend von einem Oligonukleotid, das an seinem 3'-Ende eine hairpin-Struktur aufweist; Herstellung des Gensubfragmentes HMG III-1.

0,5 nmol des Oligonukleotids HMG III-1a werden in 23 µl Wasser und 5µl 10x "Nick-Translations"-Puffer (0,5 M Tris-HCl pH 7,5, 0,1 M Magnesiumchlorid, 0,1 M DTT, 500 µg/ml BSA) aufgenommen und 5 Minuten auf 95° C erhitzt. Nachdem die Lösung auf Raumtemperatur abgekühlt ist, gibt man 20 µl dNTP-Lösung (enthaltend je 2,5 mM dATP, TTP, dCTP und dGTP) sowie 2 µl DNA-Polymerase (Klenow-Fragment, 10 units) zu und inkubiert eine Stunde bei Raumtemperatur. Nach 2, 5, 10, 20 und 60 Minuten können 1,5 µl-Proben gezogen werden, um den Verlauf der Reaktion anhand eines 10 %-igen nativen Polyacrylamidgels elektrophoretisch zu verfolgen. Als Marker dient pBR322, geschnitten mit MspI. Zur Erzeugung der überhängenden Enden werden 25 µl der DNA Polymerase-Reaktionslösung 5 Minuten auf 95° C erhitzt, auf 37° C temperiert und mit 15 µl 5x EcoRI-Puffer verdünnt. Nach der Zugabe von 12,5 µl 0,2 M NaCl, 37,5 µl Wasser, 5 µl EcoRI und 5 µl HindIII (je 100 units Enzym) inkubiert man 15 Stunden bei 37° C. Der Ansatz wird durch Elektrophorese auf einem 10 %-igen Polyacrylamidgel (ohne Harnstoff) gereinigt. Die Hauptbande, die gegen den pBR322-Marker wie ein 99 Basenpaarfragment läuft, wird ausgeschnitten und nach Homogenisierung mit 0,2 M Triethylammoniumbikarbonat-Puffer eluiert. Durch Entsalzung über eine ®Sephadex-G50-Säule erhält man das gereinigte DNA-Fragment mit den überhängenden Enden für die Restriktionsenzyme EcoRI und HindIII. Dieses Fragment wird in das mit EcoRI/HindIII geöffnete pUC18-Plasmid eingebaut. Man erhält ein Hybridplasmid, enthaltend das Gensubfragment HMG III-1. In analoger Weise läßt sich ein Hybridplasmid herstellen, das das Subfragment HMG III-3 enthält.

6. Verknüpfung eines Oligonukleotids, das an seinem 3'-Ende eine Ribonukleosid-Einheit besitzt, mit einem anderen Oligonukleotid, das an seinem 3'-Ende eine hairpin-Struktur besitzt, Herstellung des Fragments HMG IV-(3 + 4)

a) Verknüpfung der beiden Oligonukleotide mit Hilfe von T4 DNA-Ligase und dem komplementären Oligonukleotid HMG IV-3b.

Je 0,1 nmol der Oligonukleotide HMG IV-3a, HMG IV-4a (als 5'-Phosphat) und HMG IV-3b (als 5'-Phosphat) werden wie in Beispiel 3 beschrieben hybridisiert und mit T4 Polynukleotid-Ligase verknüpft, wobei bei 22°C inkubiert wird. Die Reaktion kann mit Hilfe eines 8 %-igen Polyacrylamid-Gels verfolgt werden. Zur Bildung des vollständigen zweiten Stranges gleicht man die Salzkonzentration den in Beispiel 5 angegebenen Bedingungen an, gibt die Deoxynukleosidtriphosphate und die DNA-Polymerase (Klenow) zu und verfährt weiter wie in Beispiel 5 beschrieben. Nach Restriktionsenzymverdauung mit EcoRI und XbaI nach den Maßgaben des Herstellers erhält man das Genfragment HMG IV-(3 + 4). Dieses Fragment wird in bekannter Weise in mit EcoRI/XbaI geöffnetes pUC18-Plasmid ligiert. Man erhält ein Hybridplasmid, enthaltend das Genfragment HMG IV-(3 + 4).

b) Verknüpfung der beiden Oligonukleotide mit Hilfe von T4 RNA-Ligase (ohne das komplementäre Oligonukleotid HMG IV-3a)

10 pmol des 121-mer Oligonukleotids HMG IV-4a (5'-Phosphat) werden mit 100 pmol des 86-mer Oligonukleotids HMG IV-3a in 10 µl Reaktionspuffer, enthaltend 50 mM Tris-HCl (pH 8), 10 mM Magnesiumchlorid, 10 µg/ml BSA, 25 % Polyethylenglykol 6000, 1 mM Hexammin-kobalt(III)chlorid und 20 µM ATP über Nacht (16 Stunden) in Gegenwart von T4 RNA-Ligase inkubiert. Nach erfolgter Verknüpfung wird wie in Beispiel 5 beschrieben der DNA-Doppelstrang gebildet, wobei anstelle der DNA-Polymerase (Klenow) die AMV Reverse Transkriptase verwendet wird. Durch Verdauung mit den Restriktionsenzymen EcoRI und XbaI erhält man das Genfragment HMG IV-(3 + 4).

7. Herstellung von Vektoren, die die DNA-Sequenz VI enthalten.

Die DNA-Sequenz VI wird wie in den Beispielen 1 und 4 beschrieben hergestellt. Das handelsübliche Plasmid pUC18 (bzw. pUC19, M13mp18 oder M13mp19) wird nach den Angaben des Herstellers mit den Restriktionsenzymen EcoRI/HindIII geöffnet. Der Verdauungsansatz wird auf einem 1 %-igen Agarosegel durch Elektrophorese aufgetrennt. Die durch Ethidiumbromidfärbung sichtbar gemachten Plasmidbanden werden ausgeschnitten und von der Agarose elektrophoretisch eluiert. 20 fmol des so erhaltenen Rumpfplasmids werden dann mit 200 fmol des DNA-Fragments, entsprechend der DNA-Sequenz VI, über Nacht bei Raumtemperatur ligiert. Man erhält einen neuen Klonierungsvektor pSU18 (bzw. pSU19, M13mUS18 oder M13mUS19). Im Gegensatz zu den käuflichen Ausgangsplasmiden lassen sich die neuen Plasmide mit dem Restriktionsenzym NcoI schneiden. Die Restriktionsenzyme EcoRI und HindIII schneiden die Plasmide ebenfalls nur einmal, da der über die EcoRI- und HindIII-schnittstellen eingebaute Polylinker diese ursprünglich vorhandenen Schnittstellen zerstört.

8. Herstellung von Hybridplasmiden, die die Genfragmente HMG I bis HMG IV enthalten.

a) Hybridplasmid, enthaltend das Genfragment HMG I

Analog wie in Beispiel 7 beschrieben wird das Plasmid pSU18 mit den Restriktionsenzymen EcoRI und NcoI aufgeschnitten und mit dem Genfragment I, das zuvor wie in Beispiel 3 beschrieben phosphoryliert wurde, ligiert.

b) Hybridplasmid, enthaltend das Genfragment HMG II

Aus den Hybridplasmiden mit den Gensubfragmenten HMG II-1, II-2 und II-3 werden durch Restriktionsenzymverdauung mit EcoRI/MluI bzw. MluI/BssHII oder BssHII/HindIII die Genfragmente HMG II-1 bzw. HMG II-2 oder HMG II-3 isoliert. Diese werden dann in bekannter Weise in das mit EcoRI/HindIII geöffnete pSU18-Plasmid ligiert.

c) Hybridplasmid, enthaltend das Genfragment HMG III

Aus den Hybridplasmiden mit den Gensubfragmenten HMG III-1 und III-3 werden durch Verdauung mit den Restriktionsenzymen EcoRI/HindIII und Nachschneiden mit Sau96I das Genfragment HMG III-1 bzw. mit BamHI/BanII das Genfragment HMG III-3 isoliert. Diese Fragmente lassen sich mit dem HMG III-2-Fragment in ein mit HindIII/BamHI geöffnetes pSU18-Plasmid einbauen.

d) Hybridplasmid, enthaltend das Genfragment HMG IV

Die Hybridplasmide mit den Gensubfragmenten HMG IV-(1 + 2) und IV-(3 + 4) werden mit den Restriktionsenzymen EcoRI/BamHI bzw. EcoRI/XbaI geöffnet und die Genfragmente HMG IV-(1 + 2) bzw. HMG IV-(3 + 4) durch Elektrophorese gereinigt. Die erhaltenen Fragmente werden dann in ein mit BamHI/XbaI geöffnetes pSU18-Plasmid ligiert, bei dem zuvor wie unten beschrieben mit S1-Nuklease die EcoRI-Schnittstelle zerstört wurde. Man erhält ein Hybridplasmid, das in der DNA-Sequenz IV noch eine zusätzliche AATT-Nukleotidfolge enthält. Man öffnet das Hybridplasmid an dieser Stelle durch Verdauung mit dem Restriktionsenzym EcoRI und entfernt mit S1-Nuklease die überstehenden AATT-Enden. Hierzu werden 1 µg Plasmid nach EcoRI-Verdauung mit 2 units S1-Nuklease 30 Minuten bei 20°C in 50 mM Natriumacetat-Puffer (pH 4,5), enthaltend 200 mM NaCl und 1 mM Zinkchlorid, inkubiert. Über die stumpfen Enden wird das Plasmid in bekannter Weise rezirkularisiert. Man erhält ein Hybridplasmid, das das Genfragment IV enthält.

9. Konstruktion des Hybridplasmids pUH10, das die DNA-Sequence V enthält

Das Hybridplasmid mit dem Genfragment HMG I wird mit EcoRI/HindIII geöffnet und mit dem Fragment HMG II, das durch Restriktionsenzymverdauung des entsprechenden Hybridplasmids mit EcoRI/HindIII gewonnen wird, ligiert. Das resultierende Plasmid wird dann mit HindIII/BamHI geöffnet und mit dem Fragment HMG III ligiert, das aus dem entsprechenden Plasmid mit Hilfe von HindIII/BamHI zugänglich ist. Das dabei erhaltene Plasmid wird wiederum mit BamHI/XbaI geöffnet und mit dem Fragment HMG IV verknüpft, das durch Verdauung des entsprechenden Plasmids mit BamHI/XbaI erhalten wird. Man erhält das Hybridplasmid pUH10, welches das gesamte Gen HMG, entsprechend der DNA-Sequenz V, enthält. Die Figur zeigt schematisch den Aufbau von pUH10, wobei "ori" und "Ap$^r$ " die Orientierung im Restplasmid entsprechend pUC18 andeuten.

10. Transformation der Hybridplasmide

Kompetente E. coli Zellen werden mit 0,1 bis 1 µg Plasmid transformiert und auf ampicillinenthaltenden Agarplatten plattiert. Die für die Sequenzierung verwendeten M13-Plasmide werden nach Transformation ohne Ampicillin amplifiziert. Anschließend läßt sich die Integration der Genfragmente oder des Gesamtgens HMG in den Plasmiden durch DNA-Schnellaufarbeitung bestimmen (Maniatis et al., Molecular Cloning, Cold Spring Harbor 1982).

EP 0 292 803 A2

Tabelle 1          DNA-Sequenz I   (HMG I)

------------------------------------------------------------------------

```
                                                    ─HMGI-1a─                                              ►◄
MET VAL LEU VAL THR GLN GLU PRO GLU ILE GLU LEU PRO ARG GLU PRO ARG PRO ASN GLU GLU CYS LEU GLN ILE LEU GLY ASN ALA GLU
CATG GTT CTG GTT ACC CAG GAA CCT GAA ATT GAA CTT CCG CGG GAA CCT CGG CCT AAT GAA GAA TGT CTA CAG ATC CTT GGT AAT GCA GAG
     CAA GAC CAA TGG GTC CTT GGA CTT TAA CTT GAA GGC GCC CTT GGA GCC GGA TTA CTT CTT ACA GAT GTC TAG GAA CCA TTA CGT CTC
NcoI ◄──────────────────────────────────────────────────────────────────HMG I-1b──────────────────────
```

```
                                               ─HMG I-2a─                                    ►◄
LYS GLY ALA LYS PHE LEU SER ASP ALA GLU ILE ILE GLN LEU VAL ASN ALA LYS HIS ILE PRO ALA TYR LYS LEU GLU THR LEU MET GLU
AAA GGT GCA AAG TTC CTT AGT GAC GCC GAG ATC ATC CAG TTA GTG AAT GCT AAA CAT ATC CCA GCT TAC AAG CTC GAG ACT CTG ATG GAA
TTT CCA CGT TTC AAG GAA TCA CTG CGG CTC TAG TAG GTC AAT CAC TTA CGA TTT GTA TAG GGT CGA ATG TTC GAG CTC TGA GAC TAC CTT
─────────────────────────────────────────── HMG I-2b ──────────────────────────►◄
```

```
                        ─── HMG I-3a ───────────────────────────►◄          ─── HMG I-4a ───
THR HIS GLU ARG GLY VAL SER ILE ARG ARG GLN LEU LEU SER LYS LYS LEU SER GLU PRO SER SER LEU GLN TYR LEU PRO TYR ARG ASP
ACT CAT GAG CGT GGT GTA TCG ATT CGC CGA CAG TTA CTT TCG AAG AAA CTT TCT GAA CCG AGC TCT CTC CAG TAT CTG CCT TAT CGC GAT
TGA GTA CTC GCA CCA CAT AGC TAA GCG GCT GTC AAT GAA AGC TTC TTT GAA AGA CTT GGC TCG AGA GAG GTC ATA GAC GGA ATA GCG CTA
                ─────── HMG I-3b ──────────────────────►◄
```

```
                        ►◄                            ──── HMG I-5a ────
TYR ASN TYR SER LEU VAL MET GLY ALA CYS CYS GLU ASN VAL ILE GLY TYR MET PRO ILE PRO VAL GLY VAL ALA GLY PRO LEU CYS LEU
TAT AAT TAC TCT TTG GTG ATG GGA GCC TGT TGT GAG AAT GTT ATT GGA TAT ATG CCC ATC CCT GTG GGA GTG GCA GGA CCT CTT TGC TTG
ATA TTA ATG AGA AAC CAC TAC CCT CGG ACA ACA CTC TTA CAA TAA CCT ATA TAC GGG TAG GGA CAC CCT CAC CGT CCT GGA GAA ACG AAC
─────────────── HMG I-4b ──────────────────►◄                       ─── HMG I-5b ───
```

```
────────────────────►
ASP GLU LYS
GAT GAA AAA G
CTA CTT TTT CTT AA    EcoRI
──────────────────────►
```

EP 0 292 803 A2

Tabelle 2  DNA-Sequenz II  (HMG II) ----------------------------------

EcoRI ─────────────────────────────────────── HMG II-1 ──────────────────────────────

```
     PHE GLN VAL PRO MET ALA THR THR GLU GLY CYS LEU VAL ALA SER THR ASN ARG GLY CYS ARG ALA ILE GLY LEU GLY GLY GLY ALA
  AA TTC CAG GTT CCA ATG GCA ACA ACA GAA GGT TGT CTT GTG GCT AGC ACC AAT AGA GGT TGC AGA GCG ATC GGT CTT GGT GGA GGT GCC
     G GTC CAA GGT TAC CGT TGT TGT CTT CCA ACA GAA CAC CGA TCG TGG TTA TCT CCA ACG TCT CGC TAG CCA GAA CCA CCT CCA CGG
```

MluI ─────────────────────────────────── HMG II-2 ──────────────────────────

```
  SER SER ARG VAL LEU ALA ASP GLY MET THR ARG GLY PRO VAL VAL ARG LEU PRO ARG ALA CYS ASP SER ALA GLU VAL LYS ALA TRP LEU
  AGC TCC CGA GTT CTT GCA GAT GGT ATG ACG CGT GGC CCA GTT GTG CGT CTT CCA AGG GCT TGT GAC TCT GCA GAA GTG AAA GCA TGG CTC
  TCG AGG GCT CAA GAA CGT CTA CCA TAC TGC GCA CCG GGT CAA CAC GCA GAA GGT TCC CGA ACA CTG AGA CGT CTT CAC TTT CGT ACC GAG
```

BssHII ──────────────────

─────────────── HMG II-2 ─────────────────

```
  GLU THR SER GLU GLY PHE ALA VAL ILE LYS GLU ALA PHE ASP SER THR SER ARG PHE ALA ARG LEU GLN LYS LEU HIS THR SER ILE ALA
  GAA ACA TCC GAA GGG TTC GCA GTG ATC AAG GAG GCA TTT GAC AGC ACT AGC AGA TTT GCG CGC CTT CAG AAA CTT CAT ACT AGT ATC GCT
  CTT TGT AGG CTT CCC AAG CGT CAC TAG TTC CTC CGT AAA CTG TCG TGA TCG TCT AAA CGC GCG GAA GTC TTT GAA GTA TGA TCA TAG CGA
```

─────────────────────── HMG II-3 ───────────────────────────────────

```
  GLY ARG ASN LEU TYR ILE ARG PHE GLN SER ARG SER GLY ASP ALA MET GLY MET ASN MET ILE SER LYS GLY THR GLU LYS ALA LEU SER
  GGA CGC AAC CTT TAT ATC CGT TTC CAG TCC AGA TCT GGT GAC GCA ATG GGT ATG AAC ATG ATA TCT AAG GGC ACA GAG AAA GCA CTT TCC
  CCT GCG TTG GAA ATA TAG GCA AAG GTC AGG TCT AGA CCA CTG CGT TAC CCA TAC TTG TAC TAT AGA TTC CCG TGT CTC TTT CGT GAA AGG
```

A
TTC GA    Hind III

EP 0 292 803 A2

Tabelle 2.1                    DNA-Sequenz II-1
------------------------------------------------------------------------

EcoRI ─────────────────────────────────────────────── HMG II-1a ────────
      PHE GLN VAL PRO MET ALA THR THR GLU GLY CYS LEU VAL ALA SER THR ASN ARG GLY CYS ARG ALA ILE GLY LEU GLY GLY GLY ALA
AA TTC CAG GTT CCA ATG GCA ACA ACA GAA GGT TGT CTT GTG GCT AGC ACC AAT AGA GGT TGC AGA GCG ATC GGT CTT GGT GGA GGT GCC
      G GTC CAA GGT TAC CGT TGT TGT CTT CCA ACA GAA CAC CGA TCG TGG TTA TCT CCA ACG TCT CGC TAG CCA GAA CCA CCT CCA CGG
                                                ─────────────────── HMG II-1b ────────

                                        MluI            SacI
──────────────────────────────────────────────────────────▶
SER SER ARG VAL LEU ALA ASP GLY MET THR ARG
AGC TCC CGA GTT CTT GCA GAT GGT ATG ACG CGT TGA GCT
TCG AGG GCT CAA GAA CGT CTA CCA TAC TGC GCA AC
──────────────────────────────────────────────▶


Tabelle 2.2                    DNA-Sequenz II-2
------------------------------------------------------------------------

EcoRI    MluI ──────────────────────────────────────────── HMG II-2a ──────
      PHE THR ARG GLY PRO VAL VAL ARG LEU PRO ARG ALA CYS ASP SER ALA GLU VAL LYS ALA TRP LEU GLU THR SER GLU GLY PHE ALA
AA TTC ACG CGT GGC CCA GTT GTG CGT CTT CCA AGG GCT TGT GAC TCT GCA GAA GTG AAA GCA TGG CTC GAA ACA TCC GAA GGG TTC GCA
      G TGC GCA CCG GGT CAA CAC GCA GAA GGT TCC CGA ACA CTG AGA CGT CTT CAC TTT CGT ACC GAG CTT TGT AGG CTT CCC AAG CGT
                                                ─────────────────── HMG II-2b ────────

                                        BssHII          SacI
──────────────────────────────────────────────────────────▶
VAL ILE LYS GLU ALA PHE ASP SER THR SER ARG PHE ALA ARG GLU
GTG ATC AAG GAG GCA TTT GAC AGC ACT AGC AGA TTT GCG CGC GAG CT
CAC TAG TTC CTC CGT AAA CTG TCG TGA TCG TCT AAA CGC GCG C
──────────────────────────────────────────────▶

EP 0 292 803 A2

Tabelle 2.3                                          DNA-Sequenz II-3

--------------------------------------------------------------------------------------

SacI

```
                                                    ——————— HMG II-3a ———————
      ALA ARG LEU GLN LYS LEU HIS THR SER ILE ALA GLY ARG ASN LEU TYR ILE ARG PHE GLN SER ARG SER GLY ASP ALA MET GLY
   C GCG CGC CTT CAG AAA CTT CAT ACT AGT ATC GCT GGA CGC AAC CTT TAT ATC CGT TTC CAG TCC AGA TCT GGT GAC GCA ATG GGT
TC GAG CGC GCG GAA GTC TTT GAA GTA TGA TCA TAG CGA CCT GCG TTG GAA ATA TAG GCA AAG GTC AGG TCT AGA CCA CTG CGT TAC CCA
                                                    ——————— HMG II-3b ———————
      BssHII
```

```
MET ASN MET ILE SER LYS GLY THR GLU LYS ALA LEU SER
ATG AAC ATG ATA TCT AAG GGC ACA GAG AAA GCA CTT TCC A
TAC TTG TAC TAT AGA TTC CCG TGT CTC TTT CGT GAA AGG TTC GA      HindIII
```

EP 0 292 803 A2

## Tabelle 3  DNA-Sequenz III (HMG III)

------------------------------------------------------------

HindIII ─────────────────────────────────── HMG III-1 ───────────────────

```
       LEU HIS GLU TYR PHE PRO GLU MET GLN ILE LEU ALA VAL SER GLY ASN TYR CYS THR ASP LYS LYS PRO ALA ALA ILE ASN TRP ILE
    AG CTT CAC GAG TAT TTT CCG GAA ATG CAG ATT CTG GCT GTT AGT GGT AAC TAT TGT ACT GAC AAG AAA CCT GCT GCT ATC AAT TGG ATT
       A GTG CTC ATA AAA GGC CTT TAC GTC TAA GAC CGA CAA TCA CCA TTG ATA ACA TGA CTG TTC TTT GGA CGA CGA TAG TTA ACC TAA
```

Sau96I ──────────────────────────────────── HMG III-2 ───────────────────

```
    GLU GLY ARG GLY LYS SER VAL VAL CYS GLU ALA VAL ILE PRO SER LYS VAL VAL ARG GLU VAL LEU LYS THR THR THR GLU ALA MET ILE
    GAG GGC CGT GGA AAA TCT GTT GTT TGT GAA GCT GTA ATT CCA AGC AAG GTT GTT AGA GAA GTT TTA AAG ACT ACC ACA GAG GCT ATG ATT
    CTC CCG GCA CCT TTT AGA CAA CAA ACA CTT CGA CAT TAA GGT TCG TTC CAA CAA TCT CTT CAA AAT TTC TGA TGG TGT CTC CGA TAC TAA
```

BanII ───────                       ── HMG III-3 ──────────────────

```
    GLU VAL ASN ILE ASN LYS ASN LEU VAL GLY SER ALA MET ALA GLY SER ILE GLY GLY TYR ASN ALA HIS ALA ALA ASN ILE VAL THR ALA
    GAG GTT AAT ATT AAC AAG AAT TTA GTG GGC TCT GCA ATG GCT GGT AGC ATC GGA GGC TAC AAC GCT CAT GCT GCA AAC ATT GTC ACC GCT
    CTC CAA TTA TAA TTG TTC TTA AAT CAC CCG AGA CGT TAC CGA CCA TCG TAG CCT CCG ATG TTG CGA GTA CGA CGT TTG TAA CAG TGG CGA
```

BamHI ───────────────────────────────►

```
    ILE TYR ILE ALA CYS GLY GLN ASP ALA ALA GLN ASN VAL
    ATC TAC ATT GCT TGT GGC CAG GAC GCA GCT CAG AAT GTT G
    TAG ATG TAA CGA ACA CCG GTC CTG CGT CGA GTC TTA CAA CCT AG
```

## Tabelle 3.1  DNA-Sequenz III-1

------------------------------------------------------------

HindIII ──────────────────────────────────── HMG III-1a ──────────────────

```
        LYS LEU HIS GLU TYR PHE PRO GLU MET GLN ILE LEU ALA VAL SER GLY ASN TYR CYS THR ASP LYS LYS PRO ALA ALA ILE ASN TRP
    5'AGT AAG CTT CAC GAG TAT TTT CCG GAA ATG CAG ATT CTG GCT GTT AGT GGT AAC TAT TGT ACT GAC AAG AAA CCT GCT GCT ATC AAT TGG
       TCA TTC GAA GTG CTC ATA AAA GGC CTT TAC GTC TAA GAC CGA CAA TCA CCA TTG ATA ACA TGA CTG TTC TTT GGA CGA CGA TAG TTA ACC
```

Sau96I    EcoRI

```
    ILE GLU GLY
    ATT GAG GGC CGT GAA TTC GCT T
    TAA CTC CCG GCA CTT AAG CGT T
                                3'
```

EP 0 292 803 A2

Tabelle 3.2  DNA-Sequenz III-2

HMG III-2a

Sau96

```
                                                                          VAL  VAL  ARG  GLU  VAL  LEU  LYS  THR  THR  THR  GLU  ALA  MET  ILE
        GLY  ARG  GLY  LYS  SER  VAL  VAL  CYS  GLU  ALA  VAL  ILE  PRO  SER  LYS  VAL  VAL  ARG  GLU  VAL  LEU  LYS  THR  THR  THR  GLU  ALA  MET  ILE
5' GAG |GGC  CGT  GGA  AAA  TCT  GTT  GTT  TGT  GAA  GCT  GTA  ATT  CCA  AGC  AAG  GTT  GTT  AGA  GAA  GTT  TTA  AAG  ACT  ACC  ACA  GAG  GCT  ATG  ATT
   CTC  CCG| GCA  CCT  TTT  AGA  CAA  CAA  ACA  CTT  CGA  CAT  TAA  GGT  TCG  TTC  CAA  CAA  TCT  CTT  CAA  AAT  TTC  TGA  TGG  TGT  CTC  CGA  TAC  TAA
```

BanII  BamHI

```
GLU  VAL  ASN  ILE  ASN  LYS  ASN  LEU  VAL  GLY  SER
GAG  GTT  AAT  ATT  AAC  AAG  AAT  TTA  GTG  GGC  TCT  TGG  ATC  CGC  TT
CTC  CAA  TTA  TAA  TTG  TTC  TTA  AAT  CAC  CCG  AGA  ACC  TAG| GCG  TT
```
3'


Tabelle 3.3  DNA-Sequenz III-3

HMG III-3a

EcoRI          BanII

```
        GLY  SER  ALA  MET  ALA  GLY  SER  ILE  GLY  GLY  TYR  ASN  ALA  HIS  ALA  ALA  ASN  ILE  VAL  THR  ALA  ILE  TYR  ILE  ALA  CYS  GLY
5' ATG |AAT  TCG  GGC  TCT  GCA  ATG  GCT  GGT  AGC  ATC  GGA  GGC  TAC  AAC  GCT  CAT  GCT  GCA  AAC  ATT  GTC  ACC  GCT  ATC  TAC  ATT  GCT  TGT  GGC
   TAC  TTA  AGC| CCG  AGA  CGT  TAC  CGA  CCA  TCG  TAG  CCT  CCG  ATG  TTG  CGA  GTA  CGA  CGT  TTG  TAA  CAG  TGG  CGA  TAG  ATG  TAA  CGA  ACA  CCG
```

BamHI

```
GLN  ASP  ALA  ALA  GLN  ASN  VAL  GLY  SER
CAG  GAC  GCA  GCT  CAG  AAT  GTT  GGA  TCC  GCT  T
GTC  CTG  CGT  CGA  GTC  TTA  CAA  CCT  AGG  CGT  T
```
3'

EP 0 292 803 A2

Tabelle 4          DNA-Sequenz IV   (HMG IV)
------------------------------------------------------------------------------

BamHI

```
       SER SER ASN CYS ILE THR LEU MET GLU ALA SER GLY PRO THR ASN GLU ASP LEU TYR ILE SER CYS THR MET PRO SER ILE GLU ILE
 . GA TCC TCT AAC TGT ATT ACT TTA ATG GAA GCC TCA GGT CCG ACA AAT GAA GAT TTA TAT ATC AGC TGC ACC ATG CCA TCT ATC GAG ATT
        G AGA TTG ACA TAA TGA AAT TAC CTT CGG AGT CCA GGC TGT TTA CTT CTA AAT ATA TAG TCG ACG TGG TAC GGT AGA TAG CTC TAA
```

```
 GLY THR VAL GLY GLY GLY THR ASN LEU LEU PRO GLN GLN ALA CYS LEU GLN MET LEU GLY VAL GLN GLY ALA CYS LYS ASP ASN PRO GLY
 GGT ACC GTG GGT GGT GGC ACC AAC CTT CTT CCA CAG CAG GCC TGT CTG CAA ATG TTG GGT GTT CAA GGA GCA TGC AAA GAT AAT CCT GGC
 CCA TGG CAC CCA CCA CCG TGG TTG GAA GAA GGT GTC GTC CGG ACA GAC GTT TAC AAC CCA CAA GTT CCT CGT ACG TTT CTA TTA GGA CCG
```

```
 GLU ASN ALA ARG GLN LEU ALA ARG ILE VAL CYS GLY THR VAL MET ALA GLY GLU LEU SER LEU MET ALA ALA LEU ALA ALA GLY HIS LEU
 GAA AAT GCC CGG CAA CTT GCA CGA ATT GTG TGT GGG ACC GTA ATG GCC GGC GAA TTG TCT CTT ATG GCT GCC TTG GCG GCC GGA CAT CTT
 CTT TTA CGG GCC GTT GAA CGT GCT TAA CAC ACA CCC TGG CAT TAC CGG CCG CTT AAC AGA GAA TAC CGA CGG AAC CGC CGG CCT GTA GAA
```

XbaI

```
 VAL LYS SER HIS MET ILE HIS ASN ARG SER LYS ILE ASN LEU GLN ASP LEU GLN GLY ALA CYS THR LYS LYS THR ALA --- ---
 GTC AAA TCT CAT ATG ATT CAC AAC CGT TCG AAG ATC AAT TTA CAG GAT CTG CAA GGC GCT TGC ACC AAG AAG ACA GCA TAA TAG T
 CAG TTT AGA GTA TAC TAA GTG TTG GCA AGC TTC TAG TTA AAT GTC CTA GAC GTT CCG CGA ACG TGG TTC TTC TGT CGT ATT ATC AGA TC
```

EP 0 292 803 A2

Tabelle 4.1                 DNA-Sequenz IV-1
-----------------------------------------------------------------------------------

◄—— BamHI —————————————————————————————————— HMG IV-1a ————————————————

```
     GLY SER SER ASN CYS ILE THR LEU MET GLU ALA SER GLY PRO THR ASN GLU ASP LEU TYR ILE SER CYS THR MET PRO SER ILE GLU
5' TTT GGA TCC TCT AAC TGT ATT ACT TTA ATG GAA GCC TCA GGT CCG ACA AAT GAA GAT TTA TAT ATC AGC TGC ACC ATG CCA TCT ATC GAG
   AAA CCT AGG AGA TTG ACA TAA TGA AAT TAC CTT CGG AGT CCA GGC TGT TTA CTT CTA AAT ATA TAG TCG ACG TGG TAC GGT AGA TAG CTC
```

```
          KpnI/Asp718
     ILE GLY THR
     ATT GGT ACC CGT T
3'   TAA CCA TGG GCT T
```


Tabelle 4.2                 DNA-Sequenz IV-2
-----------------------------------------------------------------------------------

```
              KpnI/Asp718
          GLY THR VAL GLY GLY GLY THR ASN LEU LEU PRO GLN GLN ALA CYS LEU GLN MET LEU GLY VAL GLN GLY ALA CYS
       C T GC GGT ACC GTG GGT GGT GGC ACC AAC CTT CTT CCA CAG CAG GCC TGT CTG CAA ATG TTG GGT GTT CAA GGA GCA TGC
       T TCG CCA TGG CAC CCA CCA CCG TGG TTG GAA GAA GGT GTC GTC CGG ACA GAC GTT TAC AAC CCA CAA GTT CCT CGT ACG
```
————————————————————————————————————————— HMG IV-2a ————————————————

```
      EcoRI
   LYS ASP ASN PRO GLY ILE
   AAA GAT AAT CCT GGA ATT CAA A
   TTT CTA TTA GGA CCT TAA GTT T  5'
```

EP 0 292 803 A2

**Tabelle 4.12**          DNA-Sequenz IV-(1+2)

---------------------------------------------------------------------

BamHI

←—————————————————————————————————————— HMG IV-(1+2) —————————————————————————————

```
      SER SER ASN CYS ILE THR LEU MET GLU ALA SER GLY PRO THR ASN GLU ASP LEU TYR ILE SER CYS THR MET PRO SER ILE GLU ILE
   GA TCC TCT AAC TGT ATT ACT TTA ATG GAA GCC TCA GGT CCG ACA AAT GAA GAT TTA TAT ATC AGC TGC ACC ATG CCA TCT ATC GAG ATT
      G AGA TTG ACA TAA TGA AAT TAC CTT CGG AGT CCA GGC TGT TTA CTT CTA AAT ATA TAG TCG ACG TGG TAC GGT AGA TAG CTC TAA
```

EcoRI

————————————————————————————————————————— HMG IV-(1+2) ←———————————————————————→

```
   GLY THR VAL GLY GLY GLY THR ASN LEU LEU PRO GLN GLN ALA CYS LEU GLN MET LEU GLY VAL GLN GLY ALA CYS LYS ASP ASN PRO GLY
   GGT ACC GTG GGT GGT GGC ACC AAC CTT CTT CCA CAG CAG GCC TGT CTG CAA ATG TTG GGT GTT CAA GGA GCA TGC AAA GAT AAT CCT GG
   CCA TGG CAC CCA CCA CCG TGG TTG GAA GAA GGT GTC GTC CGG ACA GAC GTT TAC AAC CCA CAA GTT CCT CGT ACG TTT CTA TTA GGA CC TTAA
```

## Tabelle 4.3 — DNA-Sequenz IV-3

```
                                                          ———————————— HMG IV-3a ————————————
     EcoRI
      GLU PHE GLU ASN ALA ARG GLN LEU ALA ARG ILE VAL CYS GLY THR VAL MET ALA GLY GLU LEU SER LEU MET ALA ALA LEU ALA ALA
5' TCT GAA TTC GAA AAT GCC CGG CAA CTT GCA CGA ATT GTG TGT GGG ACC GTA ATG GCC GGC GAA TTG TCT CTT ATG GCT GCC TTG GCG GCC
   AGA CTT AAG CTT TTA CGG GCC GTT GAA CGT GCT TAA CAC ACA CCC TGG CAT TAC CGG CCG CTT AAC AGA GAA TAC CGA CGG AAC CGC CGG
                                                                           3'————————— HMG IV-3b ——————————
```

```
                                                          ———————————— HMG IV-4a ————————————
GLY HIS LEU VAL LYS SER HIS MET ILE HIS ASN ARG SER LYS ILE ASN LEU GLN ASP LEU GLN GLY ALA CYS THR LYS LYS THR ALA ---
GGA CAT CTT GTC AAA TCT CAT ATG ATT CAC AAC CGT TCG AAG ATC AAT TTA CAG GAT CTG CAA GGC GCT TGC ACC AAG AAG ACA GCA TAA
CCT GTA GAA CAG TTT AGA GTA TAC TAA GTG TTG GCA AGC TTC TAG TTA AAT GTC CTA GAC GTT CCG CGA ACG TGG TTC TTC TGT CGT ATT
        ————— HMG IV-4b —————                 5'                                                                      3'
```

XbaI

```
---
TAG TCT AGA CGT T
ATC AGA TCT GCT T
```

č = ribo C

## Tabelle 4.34 — DNA-Sequenz IV-(3+4)

```
◄————————————————————————————————————— HMG IV-(3+4) —————————————————————————————————————
AATT CGA AAA TGC CCG GCA ACT TGC ACG AAT TGT GTG TGG GAC CGT AAT GGC CGG CGA ATT GTC TCT TAT GGC TGC CTT GGC GGC CGG ACA
     GCT TTT ACG GGC CGT TGA ACG TGC TTA ACA CAC ACC CTG GCA TTA CCG GCC GCT TAA CAG AGA ATA CCG ACG GAA CCG CCG GCC TGT
EcoRI
```

```
                                                                                                              XbaI
——————————————————————————————————————— HMG IV-(3+4) ———————————————————————————————————————►
TCT TGT CAA ATC TCA TAT GAT TCA CAA CCG TTC GAA GAT CAA TTT ACA GGA TCT GCA AGG CGC TTG CAC CAA GAA GAC AGC ATA ATA GT
AGA ACA GTT TAG AGT ATA CTA AGT GTT GGC AAG CTT CTA GTT AAA TGT CCT AGA CGT TCC GCG AAC GTG GTT CTT CTG TCG TAT TAT CAG ATC
```

EP 0 292 803 A2

Tabelle 5　　　　　　　　DNA-Sequenz V + Aminosäuresequenz I

---

NcoI　　　　　　　　　　　　　　　　　　10　　　　　　　　　　　　　　　　　　　20
　　MET VAL LEU VAL THR GLN GLU PRO GLU ILE GLU LEU PRO ARG GLU PRO ARG PRO ASN GLU GLU CYS LEU GLN ILE LEU GLY ASN ALA GLU
5'CATG GTT CTG GTT ACC CAG GAA CCT GAA ATT GAA CTT CCG CGG GAA CCT CGG CCT AAT GAA GAA TGT CTA CAG ATC CTT GGT AAT GCA GAG
3'　　CAA GAC CAA TGG GTC CTT GGA CTT TAA CTT GAA GGC GCC CTT GGA GCC GGA TTA CTT CTT ACA GAT GTC TAG GAA CCA TTA CGT CTC

30　　　　　　　　　　　　　　　　　　40　　　　　　　　　　　　　　　　　　50
LYS GLY ALA LYS PHE LEU SER ASP ALA GLU ILE ILE GLN LEU VAL ASN ALA LYS HIS ILE PRO ALA TYR LYS LEU GLU THR LEU MET GLU
AAA GGT GCA AAG TTC CTT AGT GAC GCC GAG ATC ATC CAG TTA GTG AAT GCT AAA CAT ATC CCA GCT TAC AAG CTC GAG ACT CTG ATG GAA
TTT CCA CGT TTC AAG GAA TCA CTG CGG CTC TAG TAG GTC AAT CAC TTA CGA TTT GTA TAG GGT CGA ATG TTC GAG CTC TGA GAC TAC CTT

60　　　　　　　　　　　　　　　　　　70　　　　　　　　　　　　　　　　80
THR HIS GLU ARG GLY VAL SER ILE ARG ARG GLN LEU LEU SER LYS LYS LEU SER GLU PRO SER SER LEU GLN TYR LEU PRO TYR ARG ASP
ACT CAT GAG CGT GGT GTA TCG ATT CGC CGA CAG TTA CTT TCG AAG AAA CTT TCT GAA CCG AGC TCT CTC CAG TAT CTG CCT TAT CGC GAT
TGA GTA CTC GCA CCA CAT AGC TAA GCG GCT GTC AAT GAA AGC TTC TTT GAA AGA CTT GGC TCG AGA GAG GTC ATA GAC GGA ATA GCG CTA

90　　　　　　　　　　　　　　　　　100　　　　　　　　　　　　　　　　110
TYR ASN TYR SER LEU VAL MET GLY ALA CYS CYS GLU ASN VAL ILE GLY TYR MET PRO ILE PRO VAL GLY VAL ALA GLY PRO LEU CYS LEU
TAT AAT TAC TCT TTG GTG ATG GGA GCC TGT TGT GAG AAT GTT ATT GGA TAT ATG CCC ATC CCT GTG GGA GTG GCA GGA CCT CTT TGC TTG
ATA TTA ATG AGA AAC CAC TAC CCT CGG ACA ACA CTC TTA CAA TAA CCT ATA TAC GGG TAG GGA CAC CCT CAC CGT CCT GGA GAA ACG AAC

120　　　　　　　　　　　　　　　　130　　　　　　　　　　　　　　　　140
ASP GLU LYS GLU PHE GLN VAL PRO MET ALA THR THR GLU GLY CYS LEU VAL ALA SER THR ASN ARG GLY CYS ARG ALA ILE GLY LEU GLY
GAT GAA AAA GAA TTC CAG GTT CCA ATG GCA ACA ACA GAA GGT TGT CTT GTG GCT AGC ACC AAT AGA GGT TGC AGA GCG ATC GGT CTT GGT
CTA CTT TTT CTT AAG GTC CAA GGT TAC CGT TGT TGT CTT CCA ACA GAA CAC CGA TCG TGG TTA TCT CCA ACG TCT CGC TAG CCA GAA CCA

```
GLY GLY ALA SER SER ARG VAL LEU ALA ASP GLY MET THR ARG GLY PRO VAL VAL ARG LEU PRO ARG ALA CYS ASP SER ALA GLU VAL LYS
GGA GGT GCC AGC TCC CGA GTT CTT GCA GAT GGT ATG ACG CGT GGC CCA GTT GTG CGT CTT CCA AGG GCT TGT GAC TCT GCA GAA GTG AAA
CCT CCA CGG TCG AGG GCT CAA GAA CGT CTA CCA TAC TGC GCA CCG GGT CAA CAC GCA GAA GGT TCC CGA ACA CTG AGA CGT CTT CAC TTT

ALA TRP LEU GLU THR SER GLU GLY PHE ALA VAL ILE LYS GLU ALA PHE ASP SER THR SER ARG PHE ALA ARG LEU GLN LYS LEU HIS THR
GCA TGG CTC GAA ACA TCC GAA GGG TTC GCA GTG ATC AAG GAG GCA TTT GAC AGC ACT AGC AGA TTT GCG CGC CTT CAG AAA CTT CAT ACT
CGT ACC GAG CTT TGT AGG CTT CCC AAG CGT CAC TAG TTC CTC CGT AAA CTG TCG TGA TCG TCT AAA CGC GCG GAA GTC TTT GAA GTA TGA

SER ILE ALA GLY ARG ASN LEU TYR ILE ARG PHE GLN SER ARG SER GLY ASP ALA MET GLY MET ASN MET ILE SER LYS GLY THR GLU LYS
AGT ATC GCT GGA CGC AAC CTT TAT ATC CGT TTC CAG TCC AGA TCT GGT GAC GCA ATG GGT ATG AAC ATG ATA TCT AAG GGC ACA GAG AAA
TCA TAG CGA CCT GCG TTG GAA ATA TAG GCA AAG GTC AGG TCT AGA CCA CTG CGT TAC CCA TAC TTG TAC TAT AGA TTC CCG TGT CTC TTT

ALA LEU SER LYS LEU HIS GLU TYR PHE PRO GLU MET GLN ILE LEU ALA VAL SER GLY ASN TYR CYS THR ASP LYS LYS PRO ALA ALA ILE
GCA CTT TCC AAG CTT CAC GAG TAT TTT CCG GAA ATG CAG ATT CTG GCT GTT AGT GGT AAC TAT TGT ACT GAC AAG AAA CCT GCT GCT ATC
CGT GAA AGG TTC GAA GTG CTC ATA AAA GGC CTT TAC GTC TAA GAC CGA CAA TCA CCA TTG ATA ACA TGA CTG TTC TTT GGA CGA CGA TAG

ASN TRP ILE GLU GLY ARG GLY LYS SER VAL VAL CYS GLU ALA VAL ILE PRO SER LYS VAL VAL ARG GLU VAL LEU LYS THR THR THR GLU
AAT TGG ATT GAG GGC CGT GGA AAA TCT GTT GTT TGT GAA GCT GTA ATT CCA AGC AAG GTT GTT AGA GAA GTT TTA AAG ACT ACC ACA GAG
TTA ACC TAA CTC CCG GCA CCT TTT AGA CAA CAA ACA CTT CGA CAT TAA GGT TCG TTC CAA CAA TCT CTT CAA AAT TTC TGA TGG TGT CTC

ALA MET ILE GLU VAL ASN ILE ASN LYS ASN LEU VAL GLY SER ALA MET ALA GLY SER ILE GLY GLY TYR ASN ALA HIS ALA ALA ASN ILE
GCT ATG ATT GAG GTT AAT ATT AAC AAG AAT TTA GTG GGC TCT GCA ATG GCT GGT AGC ATC GGA GGC TAC AAC GCT CAT GCT GCA AAC ATT
CGA TAC TAA CTC CAA TTA TAA TTG TTC TTA AAT CAC CCG AGA CGT TAC CGA CCA TCG TAG CCT CCG ATG TTG CGA GTA CGA CGT TTG TAA
```

EP 0 292 803 A2

```
330                                     340                                     350
VAL THR ALA ILE TYR ILE ALA CYS GLY GLN ASP ALA ALA GLN ASN VAL GLY SER SER ASN CYS ILE THR LEU MET GLU ALA SER GLY PRO
GTC ACC GCT ATC TAC ATT GCT TGT GGC CAG GAC GCA GCT CAG AAT GTT GGA TCC TCT AAC TGT ATT ACT TTA ATG GAA GCC TCA GGT CCG
CAG TGG CGA TAG ATG TAA CGA ACA CCG GTC CTG CGT CGA GTC TTA CAA CCT AGG AGA TTG ACA TAA TGA AAT TAC CTT CGG AGT CCA GGC


360                                     370                                     380
THR ASN GLU ASP LEU TYR ILE SER CYS THR MET PRO SER ILE GLU ILE GLY THR VAL GLY GLY GLY THR ASN LEU LEU PRO GLN GLN ALA
ACA AAT GAA GAT TTA TAT ATC AGC TGC ACC ATG CCA TCT ATC GAG ATT GGT ACC GTG GGT GGT GGC ACC AAC CTT CTT CCA CAG CAG GCC
TGT TTA CTT CTA AAT ATA TAG TCG ACG TGG TAC GGT AGA TAG CTC TAA CCA TGG CAC CCA CCA CCG TGG TTG GAA GAA GGT GTC GTC CGG


390                                     400                                     410
CYS LEU GLN MET LEU GLY VAL GLN GLY ALA CYS LYS ASP ASN PRO GLY GLU ASN ALA ARG GLN LEU ALA ARG ILE VAL CYS GLY THR VAL
TGT CTG CAA ATG TTG GGT GTT CAA GGA GCA TGC AAA GAT AAT CCT GGC GAA AAT GCC CGG CAA CTT GCA CGA ATT GTG TGT GGG ACC GTA
ACA GAC GTT TAC AAC CCA CAA GTT CCT CGT ACG TTT CTA TTA GGA CCG CTT TTA CGG GCC GTT GAA CGT GCT TAA CAC ACA CCC TGG CAT


420                                     430                                     440
MET ALA GLY GLU LEU SER LEU MET ALA ALA LEU ALA ALA GLY HIS LEU VAL LYS SER HIS MET ILE HIS ASN ARG SER LYS ILE ASN LEU
ATG GCC GGC GAA TTG TCT CTT ATG GCT GCC TTG GCG GCC GGA CAT CTT GTC AAA TCT CAT ATG ATT CAC AAC CGT TCG AAG ATC AAT TTA
TAC CGG CCG CTT AAC AGA GAA TAC CGA CGG AAC CGC CGG CCT GTA GAA CAG TTT AGA GTA TAC TAA GTG TTG GCA AGC TTC TAG TTA AAT


450                                     460                XbaI
GLN ASP LEU GLN GLY ALA CYS THR LYS LYS THR ALA --- ---
CAG GAT CTG CAA GGC GCT TGC ACC AAG AAG ACA GCA TAA TAG T
GTC CTA GAC GTT CCG CGA ACG TGG TTC TTC TGT CGT ATT ATC AGA TC
```

**Ansprüche**

1. Verfahren zur Herstellung eines Hydroxymethylglutaryl-Coenzym A-Reduktase-ähnlichen Proteins der Aminosäuresequenz gemäß Tabelle 5, dadurch gekennzeichnet, daß man ein Gen für dieses Protein synthetisiert, dieses in ein Expressionsplasmid einbaut und in einem geeigneten Wirtsorganismus das gewünschte Protein exprimiert.

2. Verfahren nach Anspruch 1, daduch gekennzeichnet, daß das Gen die DNA-Sequenz V (Tabelle 5) besitzt.

3. DNA-Sequenz I gemäß Tabelle 1.

4. DNA-Sequenz II, II-1, II-2, II-3 gemäß Tabellen 2, 2.1, 2.2 und 2.3.

5. DNA-Sequenz III, III-1, III-2, III-3 gemäß Tabellen 3, 3.1, 3.2 und 3.3.

6. DNA-Sequenz IV, IV-1, IV-2, IV-(1 + 2), IV-(3 + 4) gemäß Tabellen 4, 4.1, 4.2, 4.2, 4.3 und 4.34.

7. DNA-Sequenz V gemäß Tabelle 5.

8. Hybridplasmid, dadurch gekennzeichnet, daß es eine DNA-Sequenz nach Anspruch 3 - 7 enthält.

9. Wirtsorganismus, vorzugsweise E. coli, der ein Hybridplasmid nach Anspruch 8 enthält.

10. Protein der Aminosäuresequenz I (Tabelle 5).

Patentansprüche für folgenden Vertragsstaat : ES

1. Verfahren zur Herstellung eines Hydroxymethylglutaryl-Coenzym A-Reduktase-ähnlichen Proteins der Aminosäuresequenz gemäß Tabelle 5, dadurch gekennzeichnet, daß man ein Gen für dieses Protein synthetisiert, dieses in ein Expressionsplasmid einbaut und in einem geeigneten Wirtsorganismus das gewünschte Protein exprimiert.

2. Verfahren nach Anspruch 1, daduch gekennzeichnet, daß das Gen die DNA-Sequenz V (Tabellen 5) besitzt.

XbaI

IV    BamHI

III

HindIII

V    II

Ap^r    EcoRI

pUH10

I

pSU18

NcoI

ori